# EUROPEAN PATENT APPLICATION

(11) **EP 3 754 006 A1**
(43) Date of publication of application: **23.12.2020**
(21) Application number: 19181034.0
(22) Date of filing: 18.06.2019
(51) Int. Cl.: C11D 3/50, C11D 17/04, C11D 17/06, A61K 8/02, A61L 9/01, A61Q 13/00, D06M 13/00, C11D 7/26

(54) **AN ANHYDROUS PERFUME PARTICLE**

(71) Applicant: The Procter & Gamble Company, Cincinnati, OH 45202 (US)
(72) Inventor: BLYTH, Kevin Graham, Newcastle upon Tyne, NE12 9TS (GB); CHILTON, Ruth, Newcastle upon Tyne, NE12 9TS (GB)
(74) Representative: P&G Patent Belgium UK

(57) **Abstract**

The present invention relates to an anhydrous perfume particle comprising: (a) from 15wt% to 67wt% perfume; and (b) at least 33wt% hydrophobic carrier material selected from wax esters, wax alcohols, fatty alcohols, fatty acids, and mixtures thereof, wherein the perfume particle has a weight average particle size of from 4µm to 400µm, and wherein the perfume comprises at least 66wt% of perfume raw materials having a boiling point of at least 250°C and a clogP of below 3.0.

## Description

### FIELD OF THE INVENTION

The present invention relates to a perfume particle. The perfume particle of the present invention provides improved longevity of freshness, especially fabric odor performance.

### BACKGROUND OF THE INVENTION

Fabric treatment products such as laundry detergents and fabric enhancers typically deposit perfume onto the treated fabric to provide a freshness benefit, and especially a freshness benefit to the wet fabric. The present invention seeks to provide an improvement in the longevity of this freshness benefit, especially the longevity of the wet fabric odor performance benefit.

The inventors have found that a perfume particle that uses specific amounts of perfume, specific perfume raw materials, high levels of a specific hydrophobic carrier material and having a specific particle size can provide improved longevity of the freshness benefit, especially the longevity of the wet fabric odor performance benefit.

The inventors have found that the longevity benefit of the perfume particle can be further enhanced when the perfume particle is incorporated into a fabric treatment particle in such a manner so that the perfume particle is in the form of a discontinuous phase that is dispersed within a continuous phase of water-soluble material.

### SUMMARY OF THE INVENTION

The present invention relates to an anhydrous perfume particle comprising: (a) from 15wt% to 67wt% perfume; and (b) at least 33wt% hydrophobic carrier material selected from wax esters, wax alcohols, fatty alcohols, fatty acids, and mixtures thereof, wherein the perfume particle has a weight average particle size of from 4µm to 400µm, and wherein the perfume comprises at least 66wt% of perfume raw materials having a boiling point of at least 250°C and a clogP of below 3.0.

The present invention also relates to a treatment particle, wherein the fabric treatment particle comprises perfume particles of the above embodiment, wherein the perfume particles are in the form of a discontinuous phase that is dispersed within a continuous phase of water-soluble material.

### DETAILED DESCRIPTION OF THE INVENTION

**Anhydrous perfume particle:** The anhydrous perfume particle comprises: (a) from 15wt% to 67wt% perfume; and (b) at least 33wt% hydrophobic carrier material selected from wax esters, wax alcohols, fatty alcohols, fatty acids, and mixtures thereof, wherein the perfume particle has a weight average particle size of from 4µm to 400µm, and wherein the perfume comprises at least 66wt% of perfume raw materials having a boiling point of at least 250°C and a clogP of below 3.0.

The particle may comprise a filler material selected from clay, silica, zeolite, and mixtures thereof.

The particle may comprise colorant material.

**Perfume:** The perfume comprises at least 66wt% of perfume raw materials having a boiling point of at least 250°C and a clogP of below 3.0.

**Hydrophobic carrier material:** The hydrophobic carrier material is selected from wax esters, wax alcohols, fatty alcohols, fatty acids, and mixtures thereof.

Suitable waxes may be natural or synthetic. Suitable waxes suitable include beeswax, candellia wax, ozocerite, japan wax, carbowax, Fischer-Tropsch waxes, carnauba wax, petroleum wax, and any combination thereof. Petroleum wax includes paraffin wax, polyoelfins, amorphous and microcrystalline waxes.

**Treatment particle:** The treatment particle comprises perfume particles defined above. The perfume particles are in the form of a discontinuous phase that is dispersed within a continuous phase of water-soluble material.

The treatment particle may be a fabric-treatment particle.

**Fabric treatment particle:** The fabric-treatment particle typically comprises a fabric-treatment ingredient.

**Fabric-treatment ingredient:** Suitable fabric treatment ingredients are selected from: detersive surfactant, such as anionic detersive surfactants, non-ionic detersive surfactants, cationic detersive surfactants, zwitterionic detersive surfactants and amphoteric detersive surfactants; polymers, such as carboxylate polymers, soil release polymer, anti-redeposition polymers, cellulosic polymers and care polymers; bleach, such as sources of hydrogen peroxide, bleach activators, bleach catalysts and pre-formed peracids; photobleach, such as such as zinc and/or aluminium sulphonated phthalocyanine; enzymes, such as proteases, amylases, cellulases, lipases; zeolite builder; phosphate builder; co-builders, such as citric acid and citrate; carbonate, such as sodium carbonate and sodium bicarbonate; sulphate salt, such as sodium sulphate; silicate salt such as sodium silicate; chloride salt, such as sodium chloride; brighteners; chelants; hueing agents; dye transfer inhibitors; dye fixative agents; perfume; silicone; fabric softening agents, such as clay; flocculants, such as polyethyleneoxide; suds supressors; and any combination thereof.

**Water-soluble material:** A suitable water-soluble material is polyethylene glycol.

It may be preferred that the melting point of the hydrophobic carrier material is higher than the melting point of the polyethylene glycol.

**Fillers:** Suitable fillers that can be present in the particle include predominantly inorganic salt, preferably inorganic salts at are water-soluble. Suitable fillers include carbonate and/or sulphate salts, especially alkali metal salts of carbonate and/or sulphate such as sodium carbonate and/or sodium sulphate. Suitable fillers may also include clays, zeolites and silicas. A preferred clay is bentonite clay.

**Laundry detergent powder composition:** Typically, the laundry detergent powder composition is a fully formulated laundry detergent composition, not a portion thereof such as a spray-dried, extruded or agglomerate particle that only forms part of the laundry detergent composition. Typically, the composition comprises a plurality of chemically different particles, such as spray-dried base detergent particles and/or agglomerated base detergent particles and/or extruded base detergent particles, in combination with one or more, typically two or more, or five or more, or even ten or more particles selected from: surfactant particles, including surfactant agglomerates, surfactant extrudates, surfactant needles, surfactant noodles, surfactant flakes; phosphate particles; zeolite particles; silicate salt particles, especially sodium silicate particles; carbonate salt particles, especially sodium carbonate particles; polymer particles such as carboxylate polymer particles, cellulosic polymer particles, starch particles, polyester particles, polyamine particles, terephthalate polymer particles, polyethylene glycol particles; aesthetic particles such as coloured noodles, needles, lamellae particles and ring particles; enzyme particles such as protease granulates, amylase granulates, lipase granulates, cellulase granulates, mannanase granulates, pectate lyase granulates, xyloglucanase granulates, bleaching enzyme granulates and co- granulates of any of these enzymes, preferably these enzyme granulates comprise sodium sulphate; bleach particles, such as percarbonate particles, especially coated percarbonate particles, such as percarbonate coated with carbonate salt, sulphate salt, silicate salt, borosilicate salt, or any combination thereof, perborate particles, bleach activator particles such as tetra acetyl ethylene diamine particles and/or alkyl oxybenzene sulphonate particles, bleach catalyst particles such as transition metal catalyst particles, and/or isoquinolinium bleach catalyst particles, pre-formed peracid particles, especially coated pre-formed peracid particles; filler particles such as sulphate salt particles and chloride particles; clay particles such as montmorillonite particles and particles of clay and silicone; flocculant particles such as polyethylene oxide particles; wax particles such as wax agglomerates; silicone particles, brightener particles; dye transfer inhibition particles; dye fixative particles; perfume particles such as perfume microcapsules and starch encapsulated perfume accord particles, or pro-perfume particles such as Schiff base reaction product particles; hueing dye particles; chelant particles such as chelant agglomerates; and any combination thereof.

**Water-soluble laundry unit dose composition:** The water-soluble laundry unit dose composition may comprise the laundry detergent powder defined above, wherein the laundry detergent powder composition is enclosed by a water-soluble film.

**A fabric enhancer composition:** Suitable fabric enhancer compositions include fabric enhancer compositions wherein the particle is in the form of a discontinuous phase that is suspended within a continuous liquid phase

### EXAMPLES

### Materials

Perfume sample (100% active, and comprising at least 66wt% perfume raw materials having a boiling point of at least 250°C and a clogP of below 3.0)
Carnauba Wax ex Sigma Aldrich
LAS F lakes (CALSOFT F90), 93.2% Active

### Equipment

Laboratory oven
Speedmixer DAC150FVZ-K
IKA A 11 basic Analytical mill

### Example 1: Method of making the perfume particle

Combine the following ingredients in a container:
Perfume (10g): and
Carnauba wax (30g).

Seal the container and place in an oven set at 95°C to melt the wax.

When all of the sample is in the liquid form, mix in a speedmixer for 1 minute at 1500RPM and allow to cool.

Place the resultant perfume/wax composition into a mill and grind to the required particle size. Sieving can also be used to obtain the required particle size.

## Claims

1. An anhydrous perfume particle comprising:
(a) from 15wt% to 67wt% perfume; and
(b) at least 33wt% hydrophobic carrier material selected from wax esters, wax alcohols, fatty alcohols, fatty acids, and mixtures thereof,
wherein the perfume particle has a weight average particle size of from 4µm to 400µm, and wherein the perfume comprises at least 66wt% of perfume raw materials having a boiling point of at least 250°C and a clogP of below 3.0.

2. A perfume particle according to claim 1, wherein the particle comprises a filler material selected from clay, silica, zeolite, and mixtures thereof.

3. A perfume particle according to claim 1, wherein the particle comprises colorant material.

4. A treatment particle, wherein the fabric treatment particle comprises perfume particles according to any preceding claim, wherein the perfume particles are in the form of a discontinuous phase that is dispersed within a continuous phase of water-soluble material.

5. A treatment particle according to claim 4, wherein the water-soluble material is polyethylene glycol.

6. A treatment particle according to claim 5, wherein the melting point of the hydrophobic carrier material is higher than the melting point of the polyethylene glycol.

7. A laundry detergent powder composition comprising a particle according to any of claims 1-6.

8. A water-soluble laundry unit dose composition comprising the laundry detergent powder composition according to claim 7, wherein the laundry detergent powder composition is enclosed by a water-soluble film.

9. A water-insoluble fabric treatment dryer sheet comprising a particle according to any of claims 1-6.

10. A water-soluble fabric treatment sheet comprising a particle according to any of claims 1-6.

11. A fabric enhancer composition comprising a particle according to any of claims 1-6, wherein the particle is in the form of a discontinuous phase that is suspended within a continuous liquid phase.

12. A deodorant composition comprised a particle according to any of claims 1-6.

13. An air freshener composition comprising a particle according to any of claims 1-6.

14. A water-insoluble mop sheet, wherein the sheet comprises a particle according to any of claims 1-6.
